# EUROPEAN PATENT SPECIFICATION

(11) **EP 3 694 416 B1**
(45) Date of publication and mention of the grant of the patent: **20.07.2022**
(21) Application number: 18793015.1
(22) Date of filing: 10.10.2018
(51) Int. Cl.: A61B 7/04, G16H 40/67, A61B 5/00

(54) **SYSTEM FOR RECORDING CHEST SIGNALS AND METHOD USING SAID SYSTEM**
SYSTEM ZUR AUFZEICHNUNG VON BRUSTSIGNALEN UND VERFAHREN MIT DIESEM SYSTEM
SYSTÈME D'ENREGISTREMENT DE SIGNAUX THORACIQUES ET PROCÉDÉ D'UTILISATION DE CE SYSTÈME

(30) Priority: 10.10.2017 WO PCT/IB2017/056263
(43) Date of publication of application: 19.08.2020
(73) Proprietor: CSEM Centre Suisse d'Electronique et de Microtechnique SA - Recherche et Développement, 2007 Neuchâtel (CH)
(72) Inventor: STARKOV, Pierre, 1205 Genève (CH)
(74) Representative: P&TS SA (AG, Ltd.)
(86) International application number: PCT/IB2018/057848
(87) International publication number: WO 2019/073405

(56) References cited:
- WO-A2-2011/073879
- US-A1- 2016 045 183
- US-A1- 2017 071 565
- US-A1- 2017 112 439

## Description

### Field

The present invention concerns a system for recording chest signals of a user with improved reliability. The present invention further concerns a method for obtaining chest signals of a user using the system.

### Description of related art

Chest signals are typically recorded by a physician using a stethoscope. The physician checks the quality of the recorded chest signals by analyzing what he is hearing. When performing the recording, the physician may try to record a good signal by stabilizing the head of the stethoscope on the chest of his patient. He may also ask his patient to perform certain tasks, such as breathing with the mouth open, coughing, etc. He also insures that the stethoscope is positioned at the correct location on the chest.

A patient willing to record himself his chest sounds, has typically no idea whether the recorded sounds he gets are reliable and can be used for further analysis. During the recording process, the patient's hand holding the stethoscope might move, the patient's chest might also move. The patient may further perform inappropriate respiratory tasks. Therefore, the recorded chest sounds may be unreliable and thus useless for further analysis.

Document WO2011073879 describes a signal processing apparatus and its method of operation. The apparatus comprises a phonocardiogram interface adapted to receive a phonocardiogram signal, a processor adapted to analyze the phonocardiogram signal, and a flow control adapted to determine, whether a subsequent capture of the phonocardiogram signal according to a second set of capturing properties is likely to improve an accuracy of the determined analysis result. The apparatus measures only sounds related to heart. The apparatus is only able to collect good phonocardiogram signals when the user does not breath or move during the recording period.

Document US2017071565 discloses a system having a chest piece for fitting to human skin, a receiver unit and a distant server; said chest piece encapsulates a sound transducer for acquiring raw sound data for being communicated as data signal; said receiver, in operation, is in communication with said chest piece; said receiver is a portable device having a display unit and data processing ability, and is further connectable to a local data network or global internet; said distant server comprises computer coded instructions which, in operation, processes said data signals, stores medical information and communicates said data signals and/or medical information with at least two recipients, the recipients are able to communicate information.

Document WO2011073879 further discloses that if applicable the flow control coordinates the subsequent capture of the phonocardiogram signal according to the second set of capturing properties.

### Summary

The present invention, which is defined in the appended claims, concerns a system for recording chest signals of a user, the system comprising: a sensing unit comprising a first sensor configured for recording a chest signal from the chest of the user; a remote control device connectable with the sensing unit and configured for generating guiding information comprising a recording procedure to follow when performing the recording; a processing unit for processing the chest signal such as to pre-process the recorded chest signal, to extract features of the chest signal and to determine a confidence criterion of the chest signal; the remote control device being further configured for generating instructional information comprising information about the determined confidence criterion, and comprising an interface allowing the user, or an assistant performing the recording on the user, to initiate and/or stop the recording or initiate a further analysis of the processed chest signal; the system further comprising: a storage media configured for storing the following data: the recorded chest signal, the processed chest signal, the determined confidence criterion and said guiding information, said data building a user-specific database; a supervised database comprising said user-specific database; wherein said guiding information comprises breathing guidance information to inform the user what respiratory movement to perform during the recording of a chest signal; wherein the system is configured for using the supervised database in combination with a machine-learning classification or regression method to optimize the determination of the confidence criterion using the extracted features, and for generating said guiding information comprising breathing guidance information to inform the user what respiratory movement to perform during the recording of the chest signal; and wherein the system is further configured to store the confidence criterion and guiding information, if the confidence criterion indicates a good measured chest signal.

The present invention, which is defined in the appended claims, also concerns a method for recording chest signals of a user using the system, the method comprising:
connecting the sensing unit with the remote control device;
recording a chest signal;
using the remote control device for generating guiding information comprising a recording procedure to follow when performing the recording;
processing the measured chest signal such as to pre-process the recorded chest signal, to extract features of the chest signal and to determine a confidence criterion of the chest signal; and
using the remote control device for generating instructional information comprising information about the determined confidence criterion to initiate and/or stop the recording or initiate a further analysis of the processed chest signal;
storing the following data: the recorded chest signal, the processed chest signal, the determined confidence criterion, guiding information and building a user-specific database with said data;
building a supervised database comprising said user-specific database;
using the supervised database in combination with a machine-learning classification or regression method to optimize the determination of the confidence criterion and for generating said guiding information comprising breathing guidance information to inform the user what respiratory movement to perform during the recording of the chest signal; and
storing the confidence criterion and guiding information, if the confidence criterion indicates a good measured chest signal.

The system and method disclosed herein allows for recording lung-related chest signals. When recording lung-related signals, breathing patterns and user's movements are of importance in reducing noise artifacts in the recorded chest signal and obtaining a reliable chest signal. The present system and method is able to guide the user in his breathing pattern and the guidance can be personalized as a function to each user using the system and method.

The recorded chest signals have a good accuracy and reliability.

### Brief Description of the Drawings

The invention will be better understood with the aid of the description of an embodiment given by way of example and illustrated by the figures, in which:
Fig. 1 shows a system comprising a sensing unit and a control device for obtaining chest signals of a user, according to an embodiment;
Fig. 2 schematically represents a method for obtaining chest signals of a user using the system, according to an embodiment;
Fig. 3 shows a front view (Fig. 3a) and a rear view (Fig. 3b) of an ensemble device comprising the sensing unit attached to the control device, according to an embodiment;
Fig. 4 shows a front view (Fig. 4a) and a rear view (Fig. 4b) of the ensemble device, according to another embodiment;
Fig. 5 shows a spectrogram of a measured chest signal comprising motion patterns and crackles patterns;
Fig. 6 schematically represents the method using the supervised database in combination with the machine-learning classification or regression method in order to determine the confidence criterion and for generating the corrected guiding information.

### Detailed Description of possible embodiments

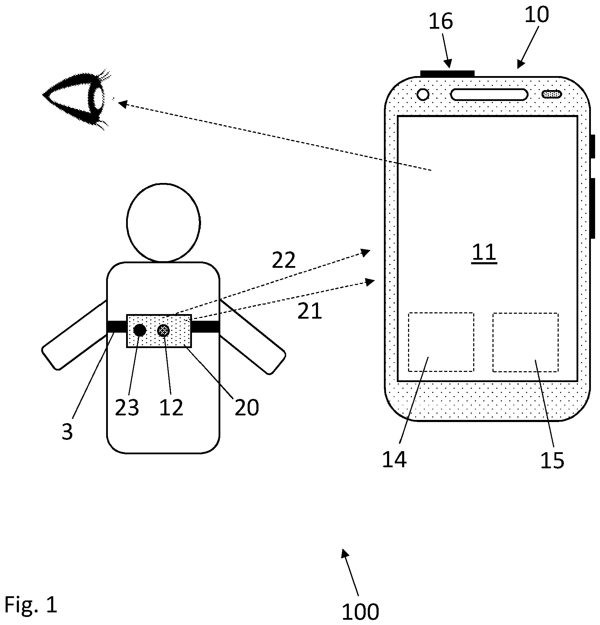

Fig. 1 shows a system 100 for obtaining chest signals of a user, according to an embodiment. The system 100 comprises a sensing unit 20 comprising a first sensor 12 configured for recording a chest signal 21. A remote control device 10 is connectable with the sensing unit 20 and configured for generating guiding information comprising a recording procedure to follow when performing the recording configured to receive the chest signal 21.

The system 100 further comprises means for processing (processing unit 15) the chest signal 21 such as to determine a confidence criterion of the chest signal 21. The remote control device 10 is further configured for generating instructional information comprising information about the determined confidence criterion. The remote control device 10 comprises an interface 11 allowing the user, or an assistant performing the recording on the user, to initiate and/or stop the recording or initiate a further analysis of the processed chest signal 21.

The first sensor 12 can be configured for recording a vibration form the user's chest (thorax) such that the chest signal 21 is a vibration signal. The first sensor 12 can be configured for measuring vibrations as sound waves with frequencies between about 20 Hz and 20 kHz. The sensing unit 20 can be configured for measuring vibrations with frequencies above 20 kHz (ultrasounds) and/or below 20 Hz (infrasound). The first sensor 12 can comprise a sound transducer such as a microphone or any suitable arrangement for registering chest sounds waves and providing a corresponding acoustic signal. In a particular embodiment, the first sensor 12 can comprise a stethoscope or an electronic stethoscope.

Alternatively, the first sensor 12 can comprise a contact microphone that senses audio vibrations through contact with the user's chest. Example of contact microphone include a microphones based on piezo materials or an accelerometer. The first sensor 12 can be a multi-sensor. The first sensor 12 can further comprise a motion sensor configured for measuring a motion signal representative of a body motion of the user.

In the particular embodiment of Fig. 1, the sensing unit 20 is comprised on a belt, or strap, 3 that is destined to be worn on the user's chest such that the sensing unit 20 located in a region of the user's chest (torso). The sensing unit 20 can also be maintained in a region of the user's chest by using a self-adhesive pad that holds the sensing unit 20 or an adhesive. The first sensor 12 comprising a contact microphone or a sound transducer can be arranged in close contact with the user's skin, possibly direct contact with the skin. The first sensor 12 can also be in contact with the user's chest through a tissue material (for example the sensing unit 20 can be placed on a t-shirt) or any other suitable materials. Such suitable materials can comprise a gel or rubber, such as a rubber ring in a stethoscope.

Positioning the sensing unit 20 on the chest with the strap 3 allows for reducing friction noise between the first sensor 12 and the chest. The strap avoid hand holding the sensing unit 20. Physiological user's hand movements can add noise to the sound recorded by the first sensor 12, thus using the strap 3 allows this type of noise to be reduced. Positioning the sensing unit 20 on the chest with the strap 3 further allows for recording chest sounds (chest signals 21) from the back of the chest. An external person is then not required for holding the sensing unit 20 in this position.

The sensing unit 20 on the chest with the strap 3 can be used on bare skin, a hairy skin and on a lightly clothed skin (i.e., t-shirt).

In an embodiment, the sensing unit 20 is configured for measuring a complementary signal 22 from the user. To that end, the sensing unit 20 can comprise a complementary sensor 23.

In particular, the complementary sensor 23 can comprise a motion sensor configured for measuring a motion signal, such that the complementary signal 22 comprises a motion signal. The motion sensor can comprise an accelerometer, a gyroscope, a magnetometer or any suitable sensor configured for measuring a motion signal representative of a body motion of the user. The complementary sensor 23 can comprise a piezoelectric sensor configured for performing ballistocardiography measurements, yielding user's body vibrations due to its cardiac and respiratory physiological signatures. Alternatively, the complementary sensor 23 can be configured for performing apex cardiography recording of low-frequency pulsations at the anterior chest wall over the apex of the heart.

The complementary sensor 23 can comprise a physiological electrical signal measuring sensor configured for measuring electrical signals, such that the complementary signal 22 comprises electrical signals. Examples of electrical signal measuring sensor comprises an electrocardiograph (ECG) device, an impedance cardiograph (ICG) device or an electromyograph (EMG) device.

The complementary sensor 23 can comprise an optical sensor configured for measuring physiological optical signals such that the complementary signal 22 comprises optical signals. In particular, the sensing unit 20 can comprise a PPG sensor or a camera for measuring visual signal.

The complementary sensor 23 can comprise a sensor configured for measuring a body temperature, for example using an infrared camera. The complementary sensor 23 can further configured for performing a respiratory measurement, for example using an airflow sensor.

The complementary sensor 23 can comprise a medical imaging device, for example including one of: ultrasonography, x-ray medical imaging, computed tomography (CT) scan, magnetic resonance imaging (MRI), positron-emission tomography (PET) scan.

The complementary sensor 23 can comprise a blood pressure sensor configured for measuring a physiological blood pressure signal, such that the complementary signal 22 comprises a blood pressure signal. Examples of blood pressure sensors include a blood pressure strap, an invasive catheter or a cuffless blood pressure monitoring device described in: "Cuffless blood pressure monitoring: CSEM's portfolio of non-occlusive technologies", Research, May 2016 (https://www.researchgate.net/publication/303487231 Cuffless blood press ure monitoring CSEM%27s portfolio of non-occlusive technologies).

The complementary sensor 23 can comprise any one of the sensors described above alone or in combination. The complementary sensor 23 can be a multi-sensor.

The complementary sensor 23 can be located in the vicinity to the first sensor 12 such that, when the sensing unit 20 contact the user, the sensing unit 20 and the complementary sensor 23 are measuring in the same area of the in the user's body. In the example of Fig. 1, both the first sensor 12 and the complementary sensor 23 are mounted close to each other on the strap 3.

However, the complementary sensor 23 need not to be in the vicinity to the first sensor 12 and can be destined to be placed on another area of the user's body. For example, in the case of an ECG and/or ICG sensor, the ECG and/or ICG electrodes can be placed at different body location that that of the first sensor 12. A PPG sensor can be placed on an extremity of the user's body, such as the fingers or earlobes.

In an embodiment, the remote control device 10 is configured for using the complementary signal 22 in combination with the chest signal 21 for determining the confidence criterion.

The complementary sensor 23 can be configured to record the complementary signal 22 independently in time with the recording of the chest signal 21. Alternatively, the complementary sensor 23 can be configured to record the complementary signal 22 substantially simultaneously with the recording of the chest signal 21. In the latter configuration, the coincidence in time between the recorded chest signal 21 and complementary signal 22 can be taken into account in determining the confidence criterion.

In the embodiment shown in Fig. 1, the sensing unit 20 is remote from the control device 10. The sensing unit 20 is then configured for transmitting the chest signal 21 and, when the system 100 comprises the complementary sensor 23, the complementary signal 21 to the control device 10. Here, the sensing unit 20 can comprise a stethoscope and the control device 10 can comprise a smartphone.

The sensing unit 20 may include a communications interface for communicating with the control device 10 via any communications means, including Wi-Fi protocol, Bluetooth protocol, 4G LTE protocol, etc. Alternatively, the sensing unit 20 may communicate with the control device 10 via a cable.

Alternatively, the sensing unit 20 and the control device 10 can be comprised in a single device to be worn by the user. Here, the system 100 can comprise an electronic stethoscope.

In another embodiment, the sensing unit 20 is attached to the control device 10 forming an ensemble device. The ensemble device can be held on the user such that the sensing unit 20 is in contact with the user's body (user's chest). Holding the ensemble device can be achieved by using a belt, a strap, a self-adhesive pad or any other fixation means. Alternatively, the ensemble device can be hand held on the user's body.

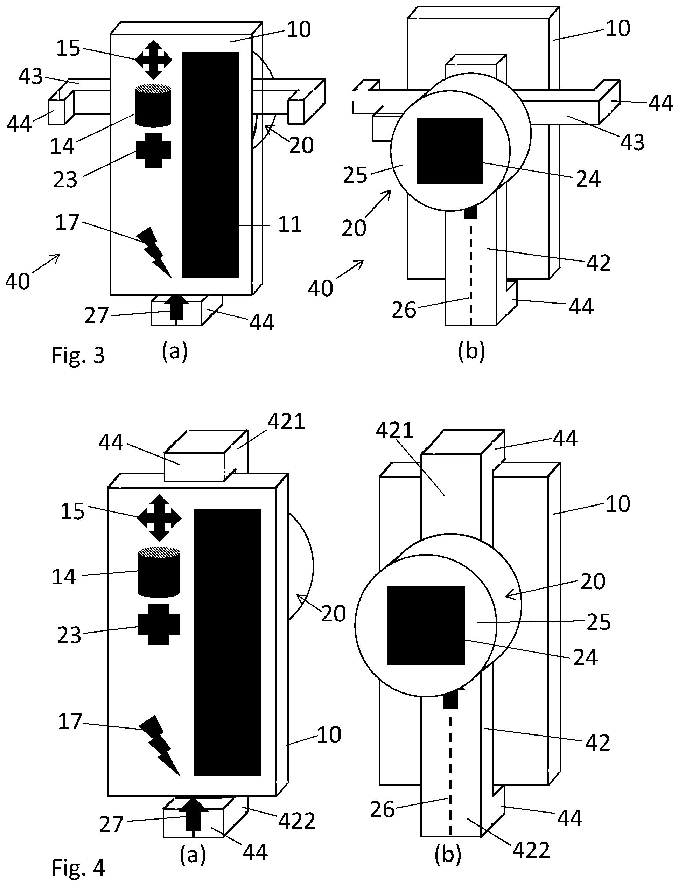

**Figs. 3 and 4** show exemplary configurations of such an ensemble device 40 wherein the control device 10 comprises the interface 11 (such as a display unit), the processing unit 15 and a power supply 17 (such as a battery). The control device 10 further comprises a complementary sensor 23 comprise a motion sensor configured for measuring a motion signal, such that the complementary signal 22 comprises a motion signal. The power supply 17 can provide power to the complementary sensor 23 and the sensing unit 20. The sensing unit 20 is removably attached to the control device 10 by using a support device 41. In the exemplary configurations of Figs. 3 and 4, the control device 10 can comprise a smartphone.

More particularly, Figs. 3a shows a front view and Fig. 3b shows a rear view of the ensemble device 40 according to a variant. The support device 41 comprises a first holding element 42 comprising a locking element 44 at one of its extremity. The support device 41 further comprises a second holding element 43 arranged substantially perpendicular to the first holding element 42 and comprising a locking element 44 at each of its extremities. The control device 10 can be received in the support device 41, on the side of the first and second holding elements 42, 43 where the locking element 44 protrude. The second holding element 43 is configured to move laterally (along a direction substantially perpendicular to the first holding element 42) such that the control device 10 is clamped between the two locking elements 44 of the second holding element 43. The locking element 44 of the first holding element 42 can also be used for retaining the control device 10. The movable second holding element 43 allows for adapting the support device 41 to the width of the control device 10.

The sensing unit 20 can be removably attached to the support device 41, on the other side of the first and second holding elements 42, 43.

In the example illustrated, the first sensor 12 of the sensing unit 20 comprises a contact microphone including a diaphragm 24 configured within a sensor casing 25 such as to vibrate when in contact with the user's body. The first sensor 12 further comprises a piston (not visible) arranged for transmitting the measured sound vibrations to a transducer (also not visible).

Figs. 4a shows a front view and Fig. 4b shows a rear view of the ensemble device 40 according to another variant. The support device 41 comprises only a first holding element 42 comprising a locking element 44 at each of its extremities. The first holding element 42 comprises an upper element 421 movable along the length of the first holding element 42, relative to a lower element 422. The control device 10 is clamped between the two locking elements 44 of the first holding element 43. The movable upper element 421 allows for adapting the support device 41 to the height of the control device 10. The sensing unit 20 can be removably attached to the support device 41, on the side of the first holding element 42 opposed to the one where the control device 10 is received.

The sensing unit 20 can be electrically connected to the sensing unit 20 by wires 26 embedded in the support device 41. A connector 27, also embedded in the in the support device 41, can be provided between the wires 26 and the control device 10. In the examples of Figs 3 and 4, the wires 26 and connector 27 are embedded in the first holding element 42. In the case the control device 10 comprises a smartphone, the audio plug or the USB connector of the smartphone can be used as the connector 27.

The support device 41 having embedded wires 26 and connector 27 is compact. Parasitic vibrations, generated by a moving loose cable connecting the sensing unit 20 to the control device 10, are avoided.

In the ensemble device configuration, the power supply 17 (battery) of the control device 10 can be used for powering the sensing unit 20 such that the latter does not require an extra battery. The wires 26 and connector 27 can be used for communicating the measured chest signal 21 to the processing unit 15 in the control device 10. Since the chest signal 21 can be sent to the processing unit 15 as an audio signal, no proprietary firmware is needed.

In the ensemble device configured as shown in Figs. 3 and 4, the display of the control device 10 is oriented opposed to the face of the sensing unit 20 contacting the user's body. The person holding the sensing unit 20 on the user's body can look at the display 11 and at the sensing unit 20 at the same time. The ensemble device can be held with one hand.

Other configurations of the support 41 are possible. For example, the first holding element 42 can comprising a locking element 44 at each of its of its extremities. In another variant, the support 41 may comprise no locking element 44. In such configuration, the sensing unit 20 can be attached on the control device 10 via the (embedded) connector 27 (such as a USB or jack connector) or any other fashion.

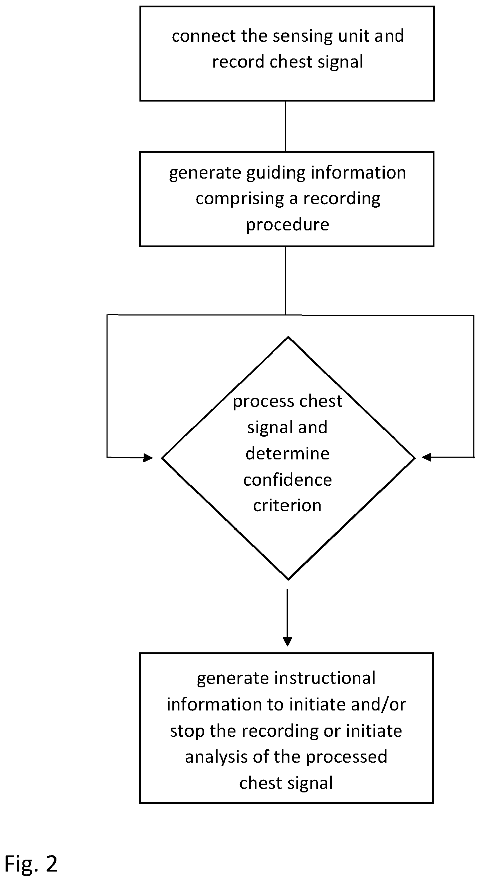

According to an embodiment schematically represented in Fig. 2, a method for obtaining chest signals of a user using the system 100 can comprise the steps of:
connecting the sensing unit 20 with the remote control device 10;
recording a chest signal 21;
using the remote control device 10 for generating guiding information comprising a recording procedure to follow when performing the recording;
processing the measured chest signal 21 such as to determine a confidence criterion of the chest signal 21; and
using the remote control device 10 for generating instructional information comprising information about the determined confidence criterion to initiate and/or stop the recording or initiate a further analysis of the processed chest signal 21.

Recording a chest signal 21 can be performed by the user from which the chest signal 21 is recorded. Alternatively, another person (an assistant) can use the system 100 to obtain chest signals 21 of a user.

Generating guiding information is typically performed prior or/and during the step of recording the chest signal 21.

In an embodiment, guiding information includes breathing guidance information destined to the user. Breathing guidance information can include any one of: how to inspire, how to expire, stop breathing, alone or in combination. Breathing guidance information can further include instructions such as: move chest without breathing, cough, etc. Breathing guidance information can further comprise instructions on movements the user should perform or whether the user should rest, during the recording.

In an embodiment, the method includes a step of generating initial information comprising a predetermined location where the user, or an assistant, should place the first sensor 12 on the user's chest. For example, predetermined locations can include different locations on the front chest and different locations on the back chest. The step of generating initial information can be performed before the step of recording the chest signal 21.

Generating instructional information is performed after the step of recording the chest signal 21, based on the processing step. Instructional information can comprise information about the determined confidence criterion.

Processing the measured chest signal 21 can comprise a step of pre-processing the recorded chest signal 21, such as normalization, filtering, formatting, etc. Processing the measured chest signal 21 further comprises a step of extracting features (for example, any type of spectral features related to a frequency analysis of the chest signal 21, such as a spectrogram) from the recorded chest signal 21. In the case features are extracted from the chest signal 21, determining the confidence criterion can comprise using the extracted feature to predict the confidence criterion.

The step of extracting features can be performed on the processed chest signal 21 being represented as a temporal / amplitude signal or as a temporal / spectral signal (such as a spectrogram or a mel-frequency cepstrum MFC).

Processing the measured chest signal 21 can be performed simultaneously with the recording time period or during a different time period. Processing the recorded chest signal 21 can be performed in the control device 10 or in a remote location (remote server).

In an embodiment, processing the measured chest signal 21 comprise using the complementary signal 22 in combination with the chest signal 21 for determining the confidence criterion.

For example, the complementary signal 22 measured from an ECG and/or an ICG sensor, i.e., the complementary sensor 23 is an ECG and/or an ICG sensor, adds heart and respiratory information to the chest signal 21.

The complementary signal 22 (motion signal) measured from a motion sensor, i.e., the complementary sensor 23 is a motion sensor (accelerometer, gyroscope or magnetometer) adds a heart and respiratory motion information to the chest signal 21.

The complementary signal 22 measured from the complementary sensor 23 comprising a PPG sensor and/or a camera focused on the chest adds respiratory and heart information to the chest signal 21.

For example, in the case the user feels chest pain (for instance due to a broken rib) he will breathe with less amplitude compared to a normal user and the chest signal 21 has a low amplitude. In that case, the complementary sensor 23 will measure a lower amplitude of the respiratory movement (for example, a lower respiratory impedance when the complementary sensor 23 is an ECG and/or an ICG sensor, a lower respiratory movement when the complementary sensor 23 is a motion sensor, etc.). The complementary signal 22 can then be used in the processing to provide an indication of the respiratory movement. Here, the lower amplitude of the respiratory movement does not indicate a wrong breathing movement of the user.

In another example, a chest signal 21 has a low amplitude is measured but the complementary signal 22 indicates that the user breathes with high amplitude. Here, the high amplitude of the respiratory movement does indicate a wrong breathing movement of the user. Thus, the complementary signal 22 is used in determining the confidence criterion.

When analyzing the measured chest signal 21 for diagnostic purposes it is important to recognize, with high accuracy, clinical patterns (such as crackles and wheezes) that are inside the chest signal 21. Indeed, diagnosis is based on detection of these patterns.

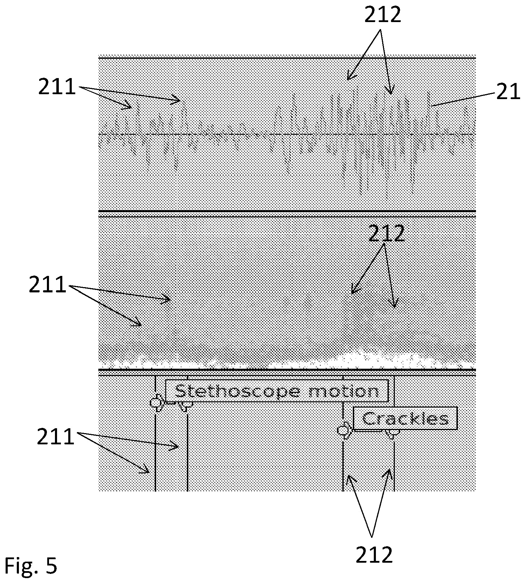

Some of the clinical patterns can be mistaken with noise that adds up to the measured chest signal 21. **Fig. 5** shows a spectrogram of an exemplary measured chest signal 21 comprising motion patterns 211 and crackles patterns 212 (mostly found in bronchitis). On the spectrogram, motion patterns 211 and crackles patterns 212 are also represented with vertical lines. Motion patterns originate from motion of the sensing unit 20 comprising the first sensor 12 (sound transducer) relative to the user's body. Crackles patterns originate from the disease. Not being able to differentiate between the two types of patterns can seriously reduce the ability to perform the correct diagnose from the measured chest signal 21. It is understood that the example of Fig. 5 can apply to any breathing pattern having or not a disease-specific auscultation sounds (e.g. crackles and wheezes) in the chest signal 21.

In an embodiment, the complementary signal 22 comprising the motion signal measured from the motion sensor comprised in the complementary sensor 23 or in the first sensor 12 is used for recording vibrations and/or any movement of the sensing unit 20 relative to the user's body. Processing the measured chest signal 21 can thus comprise using the complementary signal 22 comprising the motion signal (indicative of a motion of the sensing unit 20 relative to the user's body) in combination with the chest signal 21 such as to differentiate between the motion patterns originating from motion of the sensing unit 20 relative to the user's body from the breathing patterns. The processing the measured chest signal 21 can thus enhance diagnostic accuracy.

The complementary signal 22 information can thus be used when processing the measured chest signal 21 to help identifying heart and respiratory sounds comprised in the chest signal 21 and separate this contribution from noise.

The complementary signal 22 information used in the processing step can comprise one of the motion signal, electrical signal, optical signal or blood pressure signal alone or in combination.

The confidence criterion, determined using the extracted feature, and possibly using the complementary signal 22 in combination with the chest signal 21, allows for classifying the processed chest signal 21 as reliable, i.e. the processed chest signal 21 can be used for diagnostic purpose, or unreliable, i.e. the processed chest signal 21 cannot be used for diagnostic purpose and another recording of a chest signal 21 need be performed. The processed chest signal 21 as classified as reliable can contain some portion that are noisy.

In an embodiment, the system 100 comprises a display unit 11. The display unit 11 can be used for displaying instructional information. The display unit 11 can be used for displaying the guiding information and/or the initial information.

Alternatively or in combination, the system 100 can comprise a speaker device 16 (loudspeaker, earphones, ...) for signaling the instructional information, the guiding information and/or the initial information to the user or assistant.

The display unit 11 and/or speaker device 16 can be comprised in the remote control device 10. In case the remote control device 10 is a smartphone, the display unit 11 can be the phone display and the speaker device 16 can comprise headphones connected to the smartphone. The step of recording the chest signal 21 can be initiated and/or stopped by interacting with an interface 17 of the remote control device 10 (for example through the display interface 17 of the smartphone). The step of transmitting the process chest signal 21 to an external server can also be initiated by interacting with the remote control device 10.

The method can further comprise a step of analyzing the processed chest signal 21 that are classified as reliable.

Example of analysis can include: finding noisy parts of the processed chest signal 21 and annotate them as unusable; finding well-recorded parts of the processed chest signal 21 and annotate them with cardiorespiratory labels; use the cardiorespiratory labels to set-up a differential diagnosis procedure. The complementary signal 22 can be used when performing the analysis of the processed chest signal 21.

For example, finding noisy parts of the processed chest signal 21 and/or finding well-recorded parts of the processed chest signal 21 can be performed by using the respiratory and heart information obtained from the complementary signal 22, and/or by using the differentiation between the motion patterns originating from motion of the sensing unit 20 relative to the user's body from the breathing patterns obtained from the complementary signal 22 comprising the motion signal.

The analysis of the processed chest signal 21 can yield analysis data, for example including a quality metric, the cardiorespiratory labels and the differential diagnosis procedure.

In an embodiment, the step of analyzing is performed by an expert, such as a medical professional. To that end, the processed chest signal 21 can be transmitted to an external server. The external server may be connected to a network, such as the Internet in communication with a remote device that can be accessed by the expert. The expert may perform the analysis through listening and/or visualizing, or by any other appropriate procedure. The expert can analyze the transmitted processed chest signal 21 from a location that is remote from the system 100 and the user and also at a moment that is different from the time where the measurement is performed by the system 100.

In another embodiment, the step of analyzing is performed by a computer software expert, for example that uses supervised machine-learning classification and regression techniques. The computer analysis can be performed in the system 100, for example in the remote control device 10, or in an external server.

The step of analyzing can be initiated by the user, or the assistant, by using the interface 17.

The analyzed chest signal 21 (by the expert or by the computer software expert) and/or the analysis data can be transmitted back to the system 100, if the analysis was performed on an external server.

The analyzed chest signal 21 and/or the analysis data can be displayed in the display unit 11 and/or signaled in the speaker device 16.

In an embodiment, the system 100 comprises a storage media 14. The storage media 14 can be comprised in the sensing unit 20 (for example in an electronic stethoscope), in the remote control device 10, or in an external server.

The method can further comprise a step of storing the analyzed chest signal 21 and/or the analysis data in the storage media 14. The confidence criterion can also be stored in the storage media 14. Interaction labels, i.e., comprising initial information, guiding information and/or instructional information used for performing the recording step, can also be stored in the storage media 14.

The storage media 14 can further store not-yet analyzed processed chest signal 21 along with the corresponding confidence criterion. In other words, all recorded chest signals 21 are stored after the processing step.

Any one of the not-yet analyzed processed chest signal 21, corresponding confidence criterion, analyzed chest signal 21, complementary signals 22, interaction labels, well-recorded parts of the expert-analyzed chest signal 21 or the analysis data is used, alone or in combination, to build a user-specific database.

The database can further comprise a generic database comprising a plurality of chest signals previously recorded from the user. The generic database can further comprise a plurality of chest signals recorded from other users.

The user-specific database can be combined with the generic database.

A supervised database is built by using the user-specific database, possibly combined with the generic database.

The supervised database is used to optimize and personalize the step of processing the measured chest signal 21 and determine the confidence criterion.

Personalizing the step of processing can comprise complementing user-specific data to other users data already available, for example available from a remote server, when building the supervised database and computer software expert model that is fitted to the user.

In particular, the supervised database can be used in combination with a machine-learning classification or regression method. The machine-learning classification or regression method obtained from the supervised database can be used for determining the confidence criterion.

In particular, extracted features from the chest signal 21 are used by the machine-learning classification or regression method for determining the confidence criterion. The machine-learning classification or regression method, comprising hyperparameters, is trained on the supervised database. During the training of the machine-learning classification or regression method, its hyperparameters can be optimized through cyclic use of the combined user-specific database.

For example, the supervised database comprises measured chest signals 21 and the interaction labels (such as initial information, guiding information and/or instructional information used for performing the recording step). During the step of training of the machine-learning classification or regression method, the machine-learning classification or regression method hyperparameters are optimized to predict the correct interaction labels when the measured chest signals 21 are presented. Of course, other algorithms can be used as well.

For example, the supervised database can be used to optimize the supervised machine-learning classification and regression method. It can be further used by the computer software expert when performing the supervised machine-learning classification and regression method.

The supervised machine-learning classification and regression method can be applied to maximize the detection of parts in the processed chest signal 21 that cannot be used by the expert analysis.

Based on the confidence criterion determined by the machine-learning classification or regression method, the supervised machine-learning classification and regression method can be used for made to inform the user (for example by displaying the interaction labels on the interface 11) what respiratory movement he should perform and how he can correct this movement for the next recording step in order to improve the reliability of the recorded chest signal 21. In other words, based on the determined confidence criterion, the machine-learning classification or regression method generates corrected interaction labels, ,i.e., corrected guiding information comprising breathing guidance information to inform the user what respiratory movement to perform during the recording of the chest signal 21. Corrected interaction labels can include initial information and/or instructional information.

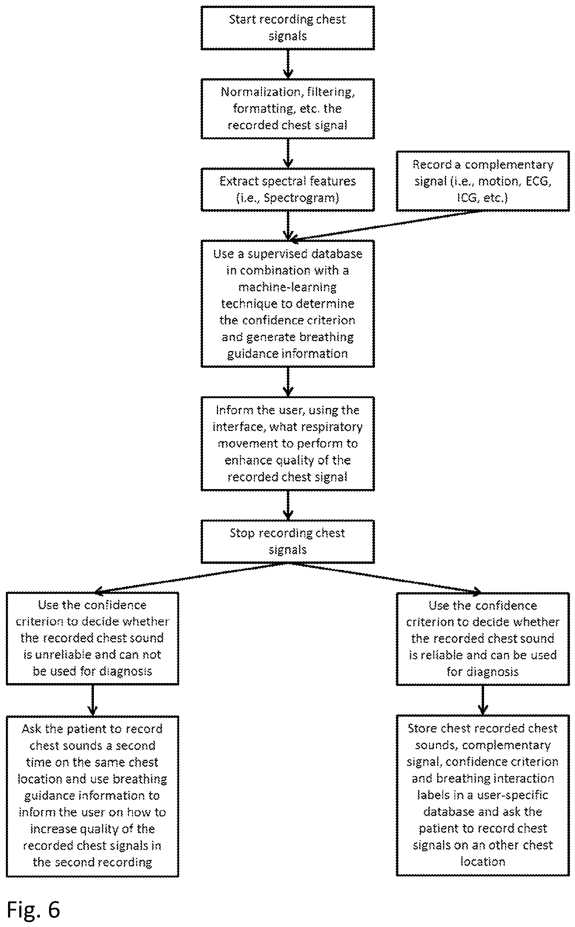

Fig. 6 schematically represents the method using the supervised database in combination with the machine-learning classification or regression method in order to determine the confidence criterion and for generating the corrected guiding information. In particular, Fig. 6 shows the steps of:
starting recording the chest signal 21;
processing the measured chest signal 21, comprising a step of pre-processing the recorded chest signal 21, such as normalization, filtering, formatting, etc., and comprising a step of extracting features (for example, any type of spectral features related to a frequency analysis of the chest signal 21, such as a spectrogram);
using a supervised database in combination with a machine-learning classification or regression method for determining the confidence criterion (using the extracted features), and for generating corrected interaction labels, ,i.e., corrected guiding information comprising breathing guidance information;
informing the user what respiratory movement to perform during the recording of the chest signal 21 (for example by displaying the interaction labels on the interface 11); and
stop recording the chest signal 21.

The complementary signal 22 information can be used in the processing step.

The method further comprises the step of using the confidence criterion to decide whether the recorded chest signal 21 is unreliable and cannot be used for diagnosis or is reliable and can be used for diagnosis.

If the recorded chest signal 21 is unreliable, the method further comprises the step of asking the user to record chest signal a second time on the same chest location and use corrected breathing guidance information to inform the user on how to increase quality of the recorded chest signal 21 in the second recording.

If the confidence criterion indicates a good measured chest signal 21, the method further comprises the step of storing the recorded chest signal 21, complementary signal 22 (if used), confidence criterion and corrected guiding information comprising breathing guidance information (breathing interaction labels) in a user-specific database and ask the patient to record chest signals on another chest location.

In an embodiment, a computer medium comprising portions of code for a software application is configured to be executed in the remote control device 10. When executed, the software application is configured for performing the method.

The user can use the system 100 according to the displayed measurement information increasing the quality of the chest signal 21 measured with the sensing unit 20.

### Reference numeral used in the figures

- 10: remote control device
- 100: system
- 11: interface, display unit
- 12: first sensor
- 14: storage media
- 15: processing unit
- 16: speaker device
- 17: power supply
- 20: sensing unit
- 21: chest signal
- 211: motion patterns
- 212: crackles patterns
- 22: complementary signal
- 23: complementary sensor
- 24: diaphragm
- 25: sensor casing
- 26: wire
- 27: connector
- 3: strap
- 40: ensemble device
- 41: support device
- 42: first holding element
- 421: upper element
- 422: lower element
- 43: second holding element
- 44: locking element

## Claims

1. A system (100) for recording chest signals of a user, the system comprising:
a sensing unit (20) comprising a first sensor (12) configured to record a chest signal (21) from the chest of the user;
a remote control device (10) connectable with the sensing unit (20) and configured to generate guiding information comprising a recording procedure to follow when performing the recording;
a processing unit (15) configured to process the chest signal (21) so as to pre-process the recorded chest signal (21), to extract features of the chest signal (21) and to use the extracted features to determine a confidence criterion of the chest signal (21);
the remote control device (10) being further configured to generate instructional information comprising information about the determined confidence criterion, and comprising an interface (11) allowing the user, or an assistant performing the recording on the user, to initiate and/or stop the recording or initiate a further analysis of the processed chest signal (21); **characterized in that**
the system (100) comprises:
a storage media (14) configured to store the following data:
the recorded chest signal (21), the processed chest signal, the determined confidence criterion and said guiding information, said data building a user-specific database;
a supervised database comprising said user-specific database;
wherein said guiding information comprises breathing guidance information to inform the user what respiratory movement to perform during the recording of a chest signal;
wherein the system (100) is configured for using the supervised database in combination with a machine-learning classification or regression method to optimize the determination of the confidence criterion using the extracted features, and for generating said guiding information comprising breathing guidance information to inform the user what respiratory movement to perform during the recording of the chest signal (21); and
wherein the system is further configured to store the recorded chest sounds, confidence criterion and guiding information in the user-specific database and to ask the patient to record chest signals on another chest location if the confidence criterion indicates a good measured chest signal (21), and to ask the patient to record chest sounds a second time on the same chest location and use the breathing guidance information to inform the user on how to increase quality of the recorded chest signals in the second recording if the confidence criterion indicates that the measured chest signal is unreliable and can not be used for diagnosis.

2. The system according to claim 1,
wherein the first sensor (12) is configured for recording a vibration from the user's chest such that the chest signal (21) is a vibration signal.

3. The system according to claim 1 or 2,
wherein the sensing unit (20) is further configured for measuring a complementary signal (22) from the user; and
wherein the processing unit (15) is configured for using the complementary signal (22) in combination with the chest signal (21) for determining the confidence criterion.

4. The system according to claim 3,
wherein the sensing unit (20) further comprises a motion sensor such that the complementary signal (22) comprises a motion signal, an electrical signal measuring sensor such that the complementary signal (22) comprises an electrical signal, or an optical sensor such that the complementary signal (22) comprises an optical signal.

5. The system according to claim 4,
wherein the electrical signal measuring sensor comprises one of an electrocardiograph (ECG), or an impedance cardiograph (ICG) or an electromyograph (EMG), a PPG sensor or a camera for measuring visual signal.

6. The system according to any one of claims 3 to 5,
wherein the sensing unit (20) further comprises a blood pressure sensor such that the complementary signal (22) comprises a blood pressure signal.

7. The system according to any one of claims 1 to 6,
wherein the interface comprises a display unit (11) or speaker device 16 (16); and
wherein the interface is further configured for signaling to the user or assistant said guiding information and instructional information.

8. Method for recording signals of a user using the system according to any one of claims 1 to 7, the method comprising:
connecting the sensing unit (20) with the remote control device (10);
recording a chest signal (21);
using the remote control device (10) for generating guiding information comprising a recording procedure to follow when performing the recording;
processing the measured chest signal (21) so as to pre-process the recorded chest signal (21), to extract features of the chest signal (21) and to use the extracted features to determine a confidence criterion of the chest signal (21); and
using the remote control device (10) for generating instructional information comprising information about the determined confidence criterion to initiate and/or stop the recording or initiate a further analysis of the processed chest signal (21);
**characterized in that** the method further comprises:
storing the following data: the recorded chest signal (21), the processed chest signal, the determined confidence criterion, guiding information and building a user-specific database with said data;
building a supervised database comprising said user-specific database;
using the supervised database in combination with a machine-learning classification or regression method to optimize the determination of the confidence criterion and for generating said guiding information comprising breathing guidance information to inform the user what respiratory movement to perform during the recording of the chest signal (21); and
storing the confidence criterion and guiding information, if the confidence criterion indicates a good measured chest signal (21), and asking the patient to record chest signals on another chest location.

9. The method according to claim 8,
wherein guiding information includes at least one of the following actions: how to inspire, how to expire, stop breathing, move chest without breathing, cough.

10. The method according to claim 8 or 9,
including a step of generating initial information comprising a predetermined location where the user, or an assistant, should place the first sensor (12) on the user's chest.

11. The method according to any one of claims 8 to 10,
wherein the step of initiating a further analysis comprises analyzing the processed chest signal (21) by a computer software expert or by an external expert.

12. The method according to any one of claims 8 to 11,
wherein the sensing unit (20) is further configured for measuring a complementary signal (22) from the user; and
wherein said processing the measured chest signal (21) uses the complementary signal (22) in combination with the chest signal (21) for determining the confidence criterion.

13. The method according to claim 12,
wherein said complementary signal (22) is used for identifying heart and respiratory sounds comprised in the chest signal (21).

14. The method according to claim 12 or 13,
wherein the complementary signal (22) comprises a motion signal indicative of a motion of the sensing unit (20) relative to the user's body; and wherein the complementary signal (22) is used in combination with the chest signal (21) for differentiating between motion patterns originating from motion of the sensing unit (20) relative to the user's body and breathing patterns.

15. Computer medium comprising portions of code for a software application configured to be executed in the processing unit (15), when executed, said software application being configured for performing the method according to any one of claims 9 to 14.

## Patentansprüche

1. System (100) zum Aufzeichnen von Brustkorbsignalen eines Benutzers, das System umfassend:
eine Erfassungseinheit (20), umfassend einen ersten Sensor (12), konfiguriert zum Aufzeichnen eines Brustkorbsignals (21) von dem Brustkorb des Benutzers;
eine Fernsteuerungsvorrichtung (10), verbindbar mit der Erfassungseinheit (20) und konfiguriert zum Erzeugen von Anleitungsinformationen, umfassend eine Aufzeichnungsprozedur, die beim Durchführen des Aufzeichnens zu befolgen ist;
eine Verarbeitungseinheit (15), konfiguriert zum Verarbeiten des Brustkorbsignals, (21), um das aufgezeichnete Brustkorbsignal (21) zu vorverarbeiten, um Merkmale des Brustkorbsignals (21) zu extrahieren und die extrahierten Merkmale zu verwenden, um ein Konfidenzkriterium des Brustkorbsignals (21) zu bestimmen;
wobei die Fernsteuerungsvorrichtung (10) ferner konfiguriert ist zum Erzeugen von Schulungsinformationen, umfassend Informationen über das bestimmte Konfidenzkriterium, und eine Schnittstelle (11) umfasst, die dem Benutzer oder einem Assistenten, der das Aufzeichnen an dem Benutzer durchführt, gestattet, das Aufzeichnen einzuleiten und/oder zu stoppen oder eine weitere Analyse des verarbeiteten Brustkorbsignals (21) einzuleiten; **dadurch gekennzeichnet, dass** das System (100) umfasst:
ein Speichermedium (14), konfiguriert zum Speichern der folgenden Daten: das aufgezeichnete Brustkorbsignal (21), das verarbeitete Brustkorbsignal, das bestimmte Konfidenzkriterium und die Anleitungsinformationen, wobei die Daten eine benutzerspezifische Datenbank erstellen;
eine überwachte Datenbank, umfassend die benutzerspezifische Datenbank;
wobei die Anleitungsinformationen Atmungsanleitungsinformationen umfassen, um den Benutzer zu informieren, welche Atembewegung er während des Aufzeichnens eines Brustkorbsignals durchführen soll;
wobei das System (100) konfiguriert ist zum Verwenden der überwachten Datenbank in Kombination mit einem Maschinenlernen-Klassifizierungs- oder Regressionsverfahren, um die Bestimmung des Konfidenzkriteriums unter Verwendung der extrahierten Merkmale zu optimieren, und zum Erzeugen der Anleitungsinformationen, umfassend Atmungsanleitungsinformationen, um den Benutzer zu informieren, welche Atembewegung er während des Aufzeichnens des Brustkorbsignals (21) durchführen soll; und
wobei das System ferner konfiguriert ist zum Speichern der aufgezeichneten Brustkorbtöne, des Konfidenzkriteriums und der Anleitungsinformationen in der benutzerspezifischen Datenbank und zum Bitten des Patienten, Brustkorbsignale an einem anderen Brustkorbort aufzuzeichnen, wenn das Konfidenzkriterium ein gutes gemessenes Brustkorbsignal (21) angibt, und zum Bitten des Patienten, Brustkorbtöne ein zweites Mal an demselben Brustkorbort aufzuzeichnen und die Atmungsanleitungsinformationen zu verwenden, um den Benutzer darüber zu informieren, wie die Qualität der aufgezeichneten Brustkorbsignale bei dem zweiten Aufzeichnen erhöht werden kann, wenn das Konfidenzkriterium angibt, dass das gemessene Brustkorbsignal unzuverlässig ist und für Diagnose nicht verwendet werden kann.

2. System nach Anspruch 1,
wobei der erste Sensor (12) konfiguriert ist zum Aufzeichnen einer Vibration von dem Brustkorb des Benutzers derart, dass das Brustkorbsignal (21) ein Vibrationssignal ist.

3. System nach Anspruch 1 oder 2,
wobei die Erfassungseinheit (20) ferner konfiguriert ist zum Messen eines Komplementärsignals (22) von dem Benutzer; und
wobei die Verarbeitungseinheit (15) konfiguriert ist zum Verwenden des Komplementärsignals (22) in Kombination mit dem Brustkorbsignal (21) zum Bestimmen des Konfidenzkriteriums.

4. System nach Anspruch 3,
wobei die Erfassungseinheit (20) ferner einen Bewegungssensor, so dass das Komplementärsignal (22) ein Bewegungssignal umfasst, einen Sensor zum Messen eines elektrischen Signals, so dass das Komplementärsignal (22) ein elektrisches Signal umfasst, oder einen optischen Sensor, so dass das Komplementärsignal (22) ein optisches Signal umfasst, umfasst.

5. System nach Anspruch 4,
wobei der Sensor zum Messen eines elektrischen Signals eines eines Elektrokardiographen (EKG) oder eines Impedanzkardiographen (ICG) oder eines Elektromyographen (EMG), eines PPG-Sensors oder einer Kamera zum Messen visueller Signale umfasst.

6. System nach einem der Ansprüche 3 bis 5,
wobei die Erfassungseinheit (20) ferner einen Blutdrucksensor umfasst, so dass das Komplementärsignal (22) ein Blutdrucksignal umfasst.

7. System nach einem der Ansprüche 1 bis 6,
wobei die Schnittstelle eine Anzeigeeinheit (11) oder Lautsprechervorrichtung (16) umfasst; und
wobei die Schnittstelle ferner konfiguriert ist, dem Benutzer oder Assistenten die Anleitungsinformationen und Schulungsinformationen zu signalisieren.

8. Verfahren zum Aufzeichnen von Signalen eines Benutzers unter Verwendung des Systems nach einem der Ansprüche 1 bis 7, das Verfahren umfassend:
Verbinden der Erfassungseinheit (20) mit der Fernsteuerungsvorrichtung (10);
Aufzeichnen eines Brustkorbsignals (21);
Verwenden der Fernsteuerungsvorrichtung (10) zum Erzeugen von Anleitungsinformationen, umfassend eine Aufzeichnungsprozedur, die beim Durchführen des Aufzeichnens zu befolgen ist;
Verarbeiten des gemessenen Brustkorbsignals (21), um das aufgezeichnete Brustkorbsignal (21) zu vorverarbeiten, Merkmale des Brustkorbsignals (21) zu extrahieren und die extrahierten Merkmale zu verwenden, um ein Konfidenzkriterium des Brustkorbsignals (21) zu bestimmen; und
Verwenden der Fernsteuerungsvorrichtung (10) zum Erzeugen von Schulungsinformationen, umfassend Informationen über das bestimmte Konfidenzkriterium, um das Aufzeichnen einzuleiten und/oder zu stoppen oder eine weitere Analyse des verarbeiteten Brustkorbsignal (21) einzuleiten;
**dadurch gekennzeichnet, dass** das Verfahren ferner umfasst:
Speichern der folgenden Daten: das aufgezeichnete Brustkorbsignal (21), das verarbeitete Brustkorbsignal, das bestimmte Konfidenzkriterium, die Anleitungsinformationen und Erstellen einer benutzerspezifischen Datenbank mit den Daten;
Erstellen einer überwachten Datenbank, die die benutzerspezifische Datenbank umfasst;
Verwenden der überwachten Datenbank in Kombination mit einem Maschinenlernen-Klassifizierungs- oder Regressionsverfahren, um die Bestimmung des Konfidenzkriteriums zu optimieren, und zum Erzeugen der Anleitungsinformationen, umfassend Atmungsanleitungsinformationen, um den Benutzer zu informieren, welche Atembewegung er während des Aufzeichnens des Brustkorbsignals (21) durchführen soll; und
Speichern des Konfidenzkriteriums und der Anleitungsinformationen, wenn das Konfidenzkriterium ein gutes gemessenes Brustkorbsignal (21) angibt, und Bitten des Patienten, Brustkorbsignale an einem anderen Brustkorbort aufzuzeichnen.

9. Verfahren nach Anspruch 8,
wobei die Anleitungsinformationen mindestens eine der folgenden Aktionen enthalten: wie einzuatmen ist, wie auszuatmen ist, Stoppen der Atmung, Bewegen des Brustkorbs ohne Atmung, Husten.

10. Verfahren nach Anspruch 8 oder 9,
enthaltend einen Schritt des Erzeugens von einleitenden Informationen, umfassend einen im Voraus bestimmten Ort, an dem der Benutzer oder ein Assistent den ersten Sensor (12) auf dem Brustkorb des Benutzers anordnen sollte.

11. Verfahren nach einem der Ansprüche 8 bis 10,
wobei der Schritt des Einleitens einer weiteren Analyse umfasst, das verarbeitete Brustkorbsignal (21) durch einen Computersoftware-Experten oder durch einen externen Experten zu analysieren.

12. Verfahren nach einem der Ansprüche 8 bis 11,
wobei die Erfassungseinheit (20) ferner konfiguriert ist zum Messen eines Komplementärsignals (22) von dem Benutzer; und
wobei das Verarbeiten des gemessenen Brustkorbsignals (21) das Komplementärsignal (22) in Kombination mit dem Brustkorbsignal (21) zum Bestimmen des Konfidenzkriteriums verwendet.

13. Verfahren nach Anspruch 12,
wobei das Komplementärsignal (22) zum Identifizieren von Herz- und Atmungstönen, die in dem Brustkorbsignal (21) enthalten sind, verwendet wird.

14. Verfahren nach Anspruch 12 oder 13,
wobei das Komplementärsignal (22) ein Bewegungssignal umfasst, das eine Bewegung der Erfassungseinheit (20) relativ zu dem Körper des Benutzers angibt; und
wobei das Komplementärsignal (22) in Kombination mit dem Brustkorbsignal (21) zum Unterscheiden zwischen Bewegungsmustern, die aus einer Bewegung der Erfassungseinheit (20) relativ zu dem Körper des Benutzers herrühren, und Atmungsmustern verwendet wird.

15. Computermedium, umfassend Codeabschnitte für eine Softwareanwendung, die konfiguriert ist, in der Verarbeitungseinheit (15) ausgeführt zu werden, wobei die Softwareanwendung, wenn sie ausgeführt wird, zum Durchführen des Verfahrens nach einem der Ansprüche 9 bis 14 konfiguriert ist.

## Revendications

1. Système (100) pour enregistrer des signaux thoraciques d'un utilisateur, le système comprenant :
une unité de détection (20) comprenant un premier capteur (12) configuré pour enregistrer un signal thoracique (21) provenant de la poitrine de l'utilisateur ;
un dispositif de commande à distance (10) pouvant être connecté à l'unité de détection (20) et configuré pour générer des informations de guidage comprenant une procédure d'enregistrement à suivre lors de la réalisation de l'enregistrement ;
une unité de traitement (15) configurée pour traiter le signal thoracique (21) de manière à prétraiter le signal thoracique enregistré (21), extraire des caractéristiques du signal thoracique (21) et utiliser les caractéristiques extraites pour déterminer un critère de confiance du signal thoracique (21) ;
le dispositif de commande à distance (10) étant en outre configuré pour générer des informations d'instruction comprenant des informations sur le critère de confiance déterminé, et comprenant une interface (11) permettant à l'utilisateur, ou à un assistant effectuant l'enregistrement sur l'utilisateur, de lancer et/ou d'arrêter l'enregistrement ou de lancer une analyse supplémentaire du signal thoracique traité (21) ;
**caractérisé en ce que** le système (100) comprend :
un support de stockage (14) configuré pour stocker les données suivantes :
le signal thoracique enregistré (21), le signal thoracique traité, le critère de confiance déterminé et
lesdites informations de guidage, lesdites données constituant une base de données spécifique à l'utilisateur ;
une base de données supervisée comprenant ladite base de données spécifique à l'utilisateur ;
où lesdites informations de guidage comprennent des informations de guidage respiratoire pour informer l'utilisateur du mouvement respiratoire à effectuer pendant l'enregistrement d'un signal thoracique ;
où le système (100) est configuré pour utiliser la base de données supervisée en combinaison avec un procédé de classification ou de régression par apprentissage machine pour optimiser la détermination du critère de confiance en utilisant les caractéristiques extraites, et pour générer lesdites informations de guidage comprenant des informations de guidage respiratoire pour informer l'utilisateur du mouvement respiratoire à effectuer pendant l'enregistrement du signal thoracique (21) ; et
où le système est en outre configuré pour stocker les sons thoraciques enregistrés, le critère de confiance et les informations de guidage dans la base de données spécifique à l'utilisateur et pour demander au patient d'enregistrer des signaux thoraciques à un autre emplacement thoracique si le critère de confiance indique un bon signal thoracique mesuré (21), et pour demander au patient d'enregistrer des sons thoraciques une deuxième fois au même emplacement thoracique et d'utiliser les informations de guidage respiratoire pour informer l'utilisateur sur la manière d'augmenter la qualité des signaux thoraciques enregistrés dans le deuxième enregistrement si le critère de confiance indique que le signal thoracique mesuré n'est pas fiable et ne peut pas être utilisé pour le diagnostic.

2. Système selon la revendication 1,
dans lequel le premier capteur (12) est configuré pour enregistrer une vibration provenant de la poitrine de l'utilisateur de sorte que le signal thoracique (21) est un signal de vibration.

3. Système selon la revendication 1 ou la revendication 2,
dans lequel l'unité de détection (20) est en outre configurée pour mesurer un signal complémentaire (22) provenant de l'utilisateur ; et
dans lequel l'unité de traitement (15) est configurée pour utiliser le signal complémentaire (22) en combinaison avec le signal thoracique (21) pour déterminer le critère de confiance.

4. Système selon la revendication 3, dans lequel l'unité de détection (20) comprend en outre un capteur de mouvement tel que le signal complémentaire (22) comprend un signal de mouvement, un capteur de mesure de signal électrique tel que le signal complémentaire (22) comprend un signal électrique, ou un capteur optique tel que le signal complémentaire (22) comprend un signal optique.

5. Système selon la revendication 4, dans lequel le capteur de mesure de signal électrique comprend l'un parmi un électrocardiographe (ECG), ou un cardiographe à impédance (ICG) ou un électromyographe (EMG), un capteur PPG ou une caméra de mesure de signal visuel.

6. Système selon l'une quelconque des revendications 3 à 5, dans lequel l'unité de détection (20) comprend en outre un capteur de pression sanguine de sorte que le signal complémentaire (22) comprend un signal de pression sanguine.

7. Système selon l'une quelconque des revendications 1 à 6, dans lequel l'interface comprend une unité d'affichage (11) ou un dispositif de haut-parleur (16) ; et
dans lequel l'interface est en outre configurée pour signaler à l'utilisateur ou à l'assistant lesdites informations de guidage et informations d'instruction.

8. Procédé d'enregistrement de signaux d'un utilisateur utilisant le système selon l'une quelconque des revendications 1 à 7, le procédé comprenant :
la connexion de l'unité de détection (20) avec le dispositif de commande à distance (10) ;
l'enregistrement d'un signal thoracique (21) ;
l'utilisation du dispositif de commande à distance (10) pour générer des informations de guidage comprenant une procédure d'enregistrement à suivre lors de la réalisation de l'enregistrement ;
le traitement du signal thoracique mesuré (21) de manière à prétraiter le signal thoracique enregistré (21), à extraire des caractéristiques du signal thoracique (21) et à utiliser les caractéristiques extraites pour déterminer un critère de confiance du signal thoracique (21) ; et
l'utilisation du dispositif de commande à distance (10) pour générer des informations d'instruction comprenant des informations sur le critère de confiance déterminé pour lancer et/ou arrêter l'enregistrement ou lancer une analyse supplémentaire du signal thoracique traité (21) ;
**caractérisé en ce que** le procédé comprend en outre :
le stockage des données suivantes : le signal thoracique enregistré (21), le signal thoracique traité, le critère de confiance déterminé, les informations de guidage et
la constitution d'une base de données spécifique à l'utilisateur avec lesdites données ;
la constitution d'une base de données supervisée comprenant ladite base de données spécifique à l'utilisateur ;
l'utilisation de la base de données supervisée en combinaison avec un procédé de classification ou de régression par apprentissage machine pour optimiser la détermination du critère de confiance et pour générer lesdites informations de guidage comprenant des informations de guidage respiratoire pour informer l'utilisateur du mouvement respiratoire à effectuer pendant l'enregistrement du signal thoracique (21) ; et
le stockage du critère de confiance et des informations de guidage, si le critère de confiance indique un bon signal thoracique mesuré (21), et la demande au patient d'enregistrer des signaux thoraciques à un autre emplacement thoracique.

9. Procédé selon la revendication 8,
dans lequel les informations de guidage comprennent au moins une des actions suivantes : comment inspirer, comment expirer, arrêter de respirer, bouger la poitrine sans respirer, tousser.

10. Procédé selon la revendication 8 ou la revendication 9, comprenant une étape de génération d'informations initiales comprenant un emplacement prédéterminé où l'utilisateur, ou un assistant, doit placer le premier capteur (12) sur la poitrine de l'utilisateur.

11. Procédé selon l'une quelconque des revendications 8 à 10, dans lequel l'étape de lancement d'une analyse supplémentaire comprend l'analyse du signal thoracique traité (21) par un logiciel informatique expert ou par un expert externe.

12. Procédé selon l'une quelconque des revendications 8 à 11, dans lequel l'unité de détection (20) est en outre configurée pour mesurer un signal complémentaire (22) de l'utilisateur ; et
dans lequel ledit traitement du signal thoracique mesuré (21) utilise le signal complémentaire (22) en combinaison avec le signal thoracique (21) pour déterminer le critère de confiance.

13. Procédé selon la revendication 12, dans lequel ledit signal complémentaire (22) est utilisé pour identifier les sons cardiaques et respiratoires compris dans le signal thoracique (21).

14. Procédé selon la revendication 12 ou la revendication 13, dans lequel le signal complémentaire (22) comprend un signal de mouvement indicatif d'un mouvement de l'unité de détection (20) par rapport au corps de l'utilisateur ; et
dans lequel le signal complémentaire (22) est utilisé en combinaison avec le signal thoracique (21) pour différencier les modèles de mouvement provenant du mouvement de l'unité de détection (20) par rapport au corps de l'utilisateur et les modèles de respiration.

15. Support informatique comprenant des parties de code pour une application logicielle configurée pour être exécutée dans l'unité de traitement (15), lorsqu'elle est exécutée, ladite application logicielle étant configurée pour exécuter le procédé selon l'une quelconque des revendications 9 à 14.
